# EUROPEAN PATENT APPLICATION

(11) **EP 2 283 809 A1**
(43) Date of publication of application: **16.02.2011**
(21) Application number: 10708880.9
(22) Date of filing: 02.03.2010
(51) Int. Cl.: A61K 9/00, A61K 38/11, A61K 38/22, A61K 38/27, A61K 38/28, A61K 47/32, A61K 47/36, A61K 47/42, A61M 37/00, A61P 3/10, A61P 5/10, A61P 7/06

(54) **MICRONEEDLE ARRAY USING POROUS SUBSTRATE AND PROCESS FOR PRODUCING SAME**

(30) Priority: 03.06.2009 JP 2009133750; 16.02.2010 JP 2010031317
(71) Applicant: BioSerenTach Co., Ltd., Kyoto 600-8040 (JP)
(72) Inventor: The designation of the inventor has not yet been filed
(74) Representative: Haley, Stephen
(86) International application number: PCT/JP2010/053338
(87) International publication number: WO 2010/140401

(57) **Abstract**

In order to produce a microneedle array which includes a macromolecular drug such as a vaccine antigen and insulin at its acral portion using a water-soluble thread-forming polymer for a base, there is a need for a substrate which enables a solvent to be efficiently removed so that a base dense solution containing a target substance can be dried and cured while still inserted in a mold. This problem is resolved by using a porous water-insoluble substrate or a porous filter as a substrate, which allows drying and curing as well as peeling from a female mold to be carried out while the base dense solution containing a target substance is still filled in the mold.

## Description

### TECHNICAL FIELD

The present invention relates to a microneedle array made of a water-soluble thread-forming polymer as the base made on a porous platform, in which a target substance is held in the acral portion of the base, and a production method thereof.

### BACKGROUND ART

Microneedles are under study as a pharmaceutical technology to improve the bioavailability of drugs having a very low bioavailability even for transdermal administration. Microneedles are fine needles that do not give pain even when inserted into the skin. As the materials for microneedles, in addition to the metals used as the conventional injection needles, microneedles made of materials such as silicon have been developed (Non-Patent Documents 1 and 2).

These microneedle arrays have the same hollow structure as injection needles, and are used for the injection of drug solution. Furthermore, self-dissolving type microneedle arrays having biosoluble substance as the base are also developed. In addition, self-dissolving type microneedles having a biosoluble substance as a base are also being developed. Where the target substance is held in the base and is administered into the epiderm followed by the self-dissolution of the base when it is inserted into the skin. For example, Patent Documents 1 to 3 and 40 to 43 disclose a self-dissolving type microneedles made of maltose as the base. There are also many known patents relating to devices and instruments for injecting a drug via microneedles, as well as the materials, shape, production method and the like of microneedles (Patent Documents 4 and 7 to 50).

The present inventor has filed a patent application directed to a microneedle having a base made of a water-soluble thread-forming polymer as a microneedle for overcoming the drawbacks of the above-described microneedles having a base made from maltose (Patent Document 5). That invention is directed to increasing the skin permeability of drugs which would not be expected to have a sufficient effect under conventional transdermal administration due to their low skin permeability, like polymeric drugs such as recombinant protein drugs, vaccines, and genetic DNA and water-soluble drugs having poor/low transdermal absorbability.

Here, the expression "water-soluble drugs having poor/low transdermal absorbability" refers to drugs which exhibit a bioavailability value of several percent or less during transdermal absorption. More specifically, this expression refers to aminoglycoside antibiotics, peptide antibiotics such as vancomycin, and vitamin C and the like.

Further, the present inventor has filed a patent application directed to an industrial production method of a microneedle array having a water-soluble thread-forming polymeric substance such as chondroitin sulfate, dextran, hyaluronic acid and the like as a base (Patent Document 6) . In addition, the present inventor has also invented a multi-layer microneedle (Patent Document 51).

In addition, the present inventor invented a method for producing a microneedle array having, for example, 100 microneedles having a length of about 500 µm and a bottom portion diameter of about 300 µm in 10 rows × 10 columns on a one square centimeter chip by using a polymer such as a vaccine antibody and a peptide/protein drug such as insulin as a target substance, and chondroitin sulfate, dextran, hyaluronic acid and the like as a base (Patent Document 52).

### PRIOR ART

### PATENT DOCUMENT

Patent Document 1: Japanese Patent Application Laid-Open No. 2003-238347
Patent Document 2: Japanese Patent Application Laid-Open No. 2005-154321
Patent Document 3: Japanese Patent Application Laid-Open No. 2005-152180
Patent Document 4: Japanese Translation of PCT International Application Publication No. 2006-500973
Patent Document 5: Pamphlet of International Publication 2006/080508
Patent Document 6: Japanese Patent Application No. 2007-150574
Patent Document 7: Japanese Translation of PCT International Application Publication No. 2006-513811 (WO 2005/044364)
Patent Document 8: Japanese Translation of PCT International Application Publication No. 2006-502831 (WO 2004/035105)
Patent Document 9: Japanese Translation of PCT International Application Publication No. 2005-533625 (WO 2004/009172)
Patent Document 10: Japanese Translation of PCT International Application Publication No. 2005-514179 (WO 2003/059431)
Patent Document 11: Japanese Translation of PCT International Application Publication No. 2005-503194 (WO 2002/100474)
Patent Document 12: Japanese Translation of PCT International Application Publication No. 2005-501615 (WO 2003/020359)
Patent Document 13: Japanese Translation of PCT International Application Publication No. 2004-532698 (WO 2002/100476)
Patent Document 14: Japanese Translation of PCT International Application Publication No. 2004-507371 (WO 2002/017985)
Patent Document 15: Japanese Translation of PCT International Application Publication No. 2004-504120 (WO 2002/007813)
Patent Document 16: Japanese Translation of PCT International Application Publication No. 2002-526273 (WO 2000/16833)
Patent Document 17: Japanese Patent Application Laid-Open No. 2008-46507
Patent Document 18: Japanese Patent Application Laid-Open No. 2008-29710
Patent Document 19: Japanese Patent Application Laid-Open No. 2008-29559
Patent Document 20: Japanese Patent Application Laid-Open No. 2008-6178
Patent Document 21: Japanese Patent Application Laid-Open No. 2007-130030
Patent Document 22: Japanese Patent Application Laid-Open No. 2006-335754
Patent Document 23: Japanese Patent Application Laid-Open No. 2005-246595
Patent Document 24: Japanese Translation of PCT International Application Publication No. 2007-523771 (WO 2005/082596)
Patent Document 25: Japanese Translation of PCT International Application Publication No. 2007-511318 (WO 2005/049107)
Patent Document 26: WO 2007/030477
Patent Document 27: WO 2004/000389
Patent Document 28: WO 2007/091608
Patent Document 29: WO 2007/116959
Patent Document 30: Japanese Patent Application Laid-Open No. 2008-011959
Patent Document 31: Japanese Patent Application Laid-Open No. 2008-074763
Patent Document 32: Japanese Patent Application Laid-Open No. 2008-125864
Patent Document 33: Japanese Patent Application Laid-Open No. 2008-237675
Patent Document 34: Japanese Patent Application Laid-Open No. 2008-296037
Patent Document 35: Japanese Patent Application Laid-Open No. 2009-061212
Patent Document 36: Japanese Patent Application Laid-Open No. 2009-0722770
Patent Document 37: Japanese Patent Application Laid-Open No. 2009-078074
Patent Document 38: Japanese Translation of PCT International Application Publication No. 2009-501066 (WO 2008/010681)
Patent Document 39: Japanese Translation of PCT International Application Publication No. 2009-502261 (WO 2007/012144)
Patent Document 40: Japanese Patent Application Laid-Open No. 2009-254756
Patent Document 41: Japanese Patent Application Laid-Open No. 2009-273872
Patent Document 42: Japanese Patent Application Laid-Open No. 2009-201956
Patent Document 43: Japanese Patent Application Laid-Open No. 2009-273772
Patent Document 44: Japanese Patent Application Laid-Open No. 2009-233170
Patent Document 45: Japanese Patent Application Laid-Open No. 2009-241357
Patent Document 46: Japanese Patent Application Laid-Open No. 2009-241358
Patent Document 47: Japanese Patent Application Laid-Open No. 2009-254876
Patent Document 48: Japanese Translation of PCT International Application Publication No. 2009-507573 (WO 2007/030477)
Patent Document 49: Japanese Translation of PCT International Application Publication No. 2009-533197 (WO 2008/004781)
Patent Document 50: Japanese Translation of PCT International Application Publication No. 2009-540984 (WO 2007/147671)
Patent Document 51: WO 2009/066763
Patent Document 52: Japanese Patent Application Laid-Open No. 2009-195583
[Non-Patent Document]

Non-Patent Document 1: D. K. Armini and C. Lui, Microfabrication technology for polycaprolactone, a biodegradable polymer, Journal of Micromechanics and Microengineering, 2000, Vol. 10, p.80 - 84
Non-Patent Document 2: M. R. Prausnitz, Microneedles for transdermal drug delivery, Advanced Drug Delivery Reviews, 2004, Vol. 56, p.581 - 587

### SUMMARY OF THE INVENTION

### PROBLEMS TO BE SOLVED BY THE INVENTION

A microneedle array was prepared by filling into a female mold produced using a silicone resin and the like a target substance as a viscous solution or a suspension using a base, and then using a substrate made from paper and the like to dry and cure the filled solution or suspension. However, to develop a microneedle array as a pharmaceutical preparation, steps such as a sterilization step or the like must be carried out. Therefore, there is a need for a substrate which has better productivity and is suited to pharmaceutical production processes such as sterilization.

Since such a microneedle array is formed with tiny needles densely packed together, when pulling the microneedle array out from the female mold during production, although the needles are adhered to the substrate, some of them can remain stuck to the female mold. For a microneedle array having in its acral portion a target substance to be inserted into a human body as a pharmaceutical, the drug dose included per microneedle array unit, specifically, the number of needles, needs to be correctly determined. Therefore, there has been the problem that when pulling out from the female mold, if the predetermined number of needles is not formed on the substrate of the microneedle array, that microneedle array cannot be used as a pharmaceutical preparation.

Further, there has also been the problem that when pulling out from the female mold, if the substrate is not removed vertically from the female mold, the microneedles can break.

It is an object of the present invention to provide a substrate suitable for a pharmaceutical preparation when producing a microneedle array containing a target substance. Especially, it is an object of the present invention to provide a substrate which can be released with the microneedles firmly fixed thereto when pulling out from a female mold, a microneedle array using this substrate, and a production method thereof.

### MEANS FOR SOLVING THE PROBLEMS

As a result of extensive research to resolve the above-described problems, the present inventor was successful in producing a microneedle array by using a water-soluble thread-forming polymer as a base, and using a substrate which is suited for, after inserting a target substance and the base into a female mold for microneedle production, efficiently drying and curing while the target substance and the base are still inserted in the female mold and being released from the female mold, thereby completing the present invention.

More specifically, the substrate for a microneedle array according to the present invention having at least one flat face is characterized by
having a protrusion on an opposite face of the flat face, and
being formed from a porous material which has a porosity of which direction is directed from the flat face to the opposite face of the flat face.

Further, the substrate is characterized by including a water-insoluble fibrous polymer and an adhesive polymer.

In addition, the fibrous polymer includes at least one of crystalline cellulose, ethyl cellulose, cellulose acetate and derivatives thereof, and chitin and derivatives thereof.

Still further, the adhesive polymeric substance includes at least one of a carboxy vinyl polymer, a carboxymethyl cellulose sodium, a polyvinyl alcohol, and a hydroxy propyl cellulose.

Moreover, the porous material may be a porous polymer resin. Further, this porous polymer resin includes at least one of polyethylene, poly(methyl methacrylate), polyvinyl chloride, acrylonitrile, chlorinated polyethylene-styrene, polyoxyethylene, and polypropylene. In addition, the porous material may be a porous metal.

Still further, the microneedle array according to the present invention is characterized by comprising:
any of the above-described porous substrates;
a protruding base portion made of a thread-forming polymer material which is fixed to the substrate; and
a target substance portion provided on a acral portion of the protruding base portion.

Moreover, a method for producing a microneedle array according to the present invention, comprises the steps of:
filling a target substance and the like into a female mold;
further filling a base into the female mold;
pressing a porous substrate against the female mold to dry and cure; and
peeling the female mold from the substrate.

When removing the base remaining on the surface of the female mold with a squeegee after filling the base into the female mold, the method may further comprise a step of coating a glue on the substrate before the step of pressing the substrate to dry and cure. Further, the glue may be coated on the side faces of the substrate in addition to the surface thereof.

The step of pressing the substrate against the female mold to dry and cure may be carried out under a reduced-pressure environment. Usually, the method is carried out under a room temperature to low-temperature environment.

### ADVANTAGES OF THE INVENTION

A microneedle array serving as a pharmaceutical preparation has to be produced using a pharmaceutical additive or undergo sterilization and the like during the production process. If the substrate of the present invention is used, after filling a female mold for microneedle array production with a viscous base dense solution or viscous suspension including a target substance, the solution can be dried and cured while the substrate is still pressed against the female mold, and then it is peeled from the female mold. Consequently, a microneedle array can be efficiently produced.

Further, since the microneedle array is produced using a thread-forming polymer as a base or as a glue in a porous substrate, during drying and curing, the base of the thread-forming polymer diffuses into the porous substrate, so that the microneedles are solidly fixed to the substrate by an anchor effect. Consequently, when peeling the substrate from the female mold, the substrate can be pulled out with all of the needles still fixed to the substrate.

In addition, since a protrusion is provided on the rear face of the porous substrate, the surface area of the rear face increases, which makes the drying and curing easier. Moreover, by gripping the protrusion on the rear face of the porous substrate with a jig, the substrate can be vertically removed from the female mold. This allows breaking of the microneedles when peeling the substrate from the female mold to be avoided. Consequently, a microneedle array can be produced formed with all of the planned microneedles arranged on the substrate.

### BRIEF DESCRIPTION OF THE DRAWINGS

Fig. 1 is a diagram illustrating, among the steps for producing the microneedle array according to the present invention, the step of filling a target substance and the like into a female mold.
Fig. 2 is a diagram illustrating a step of filling a base solution after the target substance and the like has been filled.
Fig. 3 is a diagram illustrating a step of covering with the substrate and drying and curing while pressing the substrate on the female mold after the target substance and then the base solution have been filled into the female mold.
Fig. 4 is a schematic diagram illustrating a state in which a base made of a thread-forming polymer diffuses into the substrate.
Fig. 5 is a diagram illustrating examples of variations of a protrusion formed on a rear face of the substrate.
Fig. 6 is a schematic diagram illustrating a state in which a protrusion is gripped to lift a substrate vertically upward from a female mold.
Fig. 7 is a diagram illustrating a completed microneedle array.
Fig. 8 illustrates a state in which, when a glue made from a thread-forming polymer is coated on only one face of a substrate produced from only a fibrous polymer, the substrate is warped due to excessive drying, and a state in which a glue made from a thread-forming polymeric substance was also coated on the sides of the substrate in order to resolve this problem.

### BEST MODE FOR CARRYING OUT THE INVENTION

The substrate for a microneedle array used in the present invention is a water-insoluble porous substrate. Therefore, after a water-soluble thread-forming polymer base containing a target substance is filled to the female mold of the microneedle array, the substrate can be dried and cured while pressing the substrate against the female mold.

Further, the porous substrate used in the present invention may be formed by molding a water-insoluble material. In addition, a porous filter produced from a known polymer resin or metal may also be used. The porous substrate of the present invention used in the microneedle array can be reliably peeled from the female mold with the needles fixed to the substrate by an anchor effect as a result of diffusing and curing the base made of a thread-forming polymeric substance during drying in the substrate. Therefore, it is enough for the "porosity" of the substrate of the present invention to be at a level which allows a thread-forming polymer to diffusee and be dried.

Preferably, the water-insoluble material (also referred to as "water-insoluble polymer") is a tablet produced using cellulose acetate, crystalline cellulose, ethyl cellulose, cellulose derivatives, and chitin and derivatives thereof. These substances may also be called a " fibrous polymer".

In addition, preferably, the porous polymer resin material is polyethylene, poly(methyl methacrylate), polyvinyl chloride, acrylonitrile-chlorinated polyethylene-styrene, polyoxyethylene, or polypropylene. In addition, the substrate of the present invention using a porous polymer may have been solidified to an extent that it has porosity by pressure molding of these polymers.

The shape of the substrate is not specifically limited, but a plate-shape having at least one flat face is preferred. This is because to adhere the microneedles, which were formed by flowing a substance into the female mold, to the flat face and remove them, a force is uniformly applied to all of the microneedles, so that no microneedles are left behind.

A protrusion is formed on a rear side of the flat face of the substrate of the present invention. The substrate pressed against the female mold has to be pulled out vertically upwards with respect to the female mold. This is because if the substrate is pulled out at an angle, the microneedles can bump into an edge of the concave portion of the female mold, and the needle portions can be damaged. Therefore, if a protrusion which can be gripped during removal is provided on the rear side of the substrate, the substrate can be easily pulled out from the female mold in a vertical direction.

Further, by forming a protrusion on the rear face of the substrate, the surface area of the rear face increases, so that drying and curing are easier when the substrate is pressed against the female mold.

The shape of the protrusion is not specifically limited. The protrusion may be formed only in the center of the substrate, or formed from one edge of the substrate to the other edge. Further, the protrusion may be formed by forming a concavity on the rear face of the substrate.

Preferably, the base made of a water-soluble thread-forming polymer is at least one substance selected from the group consisting of thread-forming polysaccharides, proteins, polyvinyl alcohols, carboxy vinyl polymers, and sodium polyacrylate. One kind of such a substance may be used, or a combination of a plurality of kinds may be used.

Preferably, the thread-forming polysaccharide is at least one substance selected from sodium chondroitin sulfate, dextran, dextran sulfate, hyaluronic acid, cyclodextrin, hydroxypropyl cellulose, alginic acid, agarose, pullulan, and glycogen, and derivatives thereof.

Preferably, the above-described thread-forming protein is at least one substance selected from serum albumin, serum α acidic glycoprotein, collagen, and gelatin, and derivatives thereof.

The method for holding the target substance in the base is not specifically limited, and various methods maybe applied. For example, by including the target substance as a supermolecule in the base, the target substance may be inserted into the female mold while held in the base. Then, using the substrate of the present invention, the solvent may be absorbed and evaporated to dry and cure the base to produce a microneedle array. Another method is to charge a target substance which has been micropulverized and formulated in dissolved base, and fill the resultant product as a suspension into the female mold. Then, using the substrate of the present invention, the solvent may be absorbed and evaporated to dry and cure the base so that a microneedle array can be released.

Preferably, the target substance is an active substance used in drugs and cosmetics.

Preferably, such a drug can be classified as a vaccine antigen, a peptide, a protein, a nucleic acid, or a polysaccharide.

Target substances in the field of cosmetics include substances for the purpose of prevention and treatment of skin whitening, anti-aging and the like.

The length of the microneedles constituting the microneedle array is not specifically limited, but is preferably about 200 to 700 micrometers, and more preferably about 300 to 600 micrometers.

The area of the substrate is not specifically limited, but is preferably about 25 square centimeters, more preferably about 5.0 square centimeters, and even more preferably about 2.0 to 1.0 square centimeters. Further, the thickness is also not specifically limited, but is preferably about 5 millimeters, and more preferably about 3 to 1 millimeters.

Next, the method for producing the microneedle array according to the present invention will be described using the drawings. The microneedle array according to the present invention is formed by filling into a female mold having a needle shape, in order, a dense solution comprising a mixture of a target substance and a base, and a viscous base dense solution, then drying and curing, and transferring the dried and cured product to a substrate. It is preferred that the female mold is a material which is not soluble in a polar solvent. This is because the microneedle array according to the present invention is based on the assumption that it will be used in a pharmaceutical, and the target substance and the base often dissolve in a polar solvent (in particular water).

The target substance filled in the acral portions of the needles does not have to include a base. Further, the base to be mixed with the target substance does not have to be the same kind of base as that which is filled after the target substance and the like is filled. In addition, in the present specification, the target substance may be a substance which has a pharmacological effect, a complex thereof, or a mixture with a substance which can become a base.

It is preferred that the female mold is a material which can be easily worked. This is because, as described above, the microneedle array has a size of about a few hundred micrometers, and thus a concavity of such a size has to be formed in the female mold. Specifically, a polymer resin such as silicone and the like, and a rubber can be preferably employed. A needle-like concavity having a depth of about 500 µm and a surface opening diameter of about 300 µm is formed in the female mold. This is because the female mold is used for forming a tiny needle-like structure having a height of about 500 µm and a bottom-face diameter of about 300 µm.

Further, this concavity is built, for example, at a density of about 100 concavities of 10 rows × 10 columns on a one square centimeter chip. Since a microneedle array is often provided as a sheet several square centimeters in size, the concavities at the above-described concavity density are formed for each surface area corresponding to one microneedle array unit. A region in which concavities are not formed may be formed around the periphery thereof. This is because many microneedle arrays can be produced with one female mold. In addition, this is because the number of needles to be planted per microneedle array can be determined, thereby allowing the amount of included target substance to be correctly set.

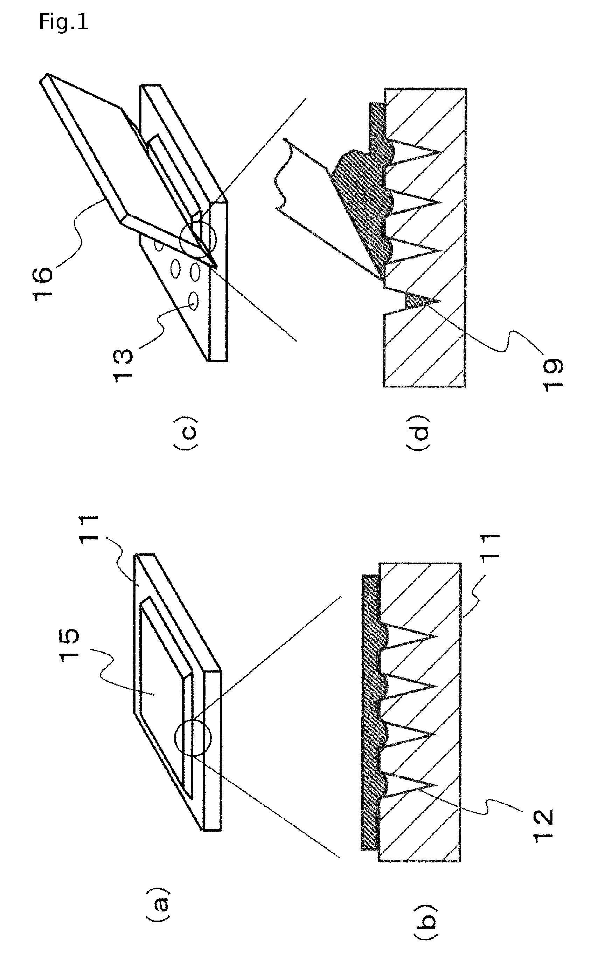

Fig. 1 illustrates the step of filling a target substance and the like into a female mold. Concavities 12 are formed in a female mold 11 at a predetermined density per unit 13 of the microneedle array. As illustrated in Figs. 1(a) and 1(c), a solution 15 containing a target substance and the like is placed in the female mold 11, and a squeegee 16 is pressed against the concavities 12 while applying pressure. Fig. 1(b) illustrates an enlarged cross-sectional view of a state in which the solution 15 containing a target substance and the like is coated.
Fig. 1(d) illustrates a cross-section of a state 19 in which the solution 15 containing a target substance and the like has been pushed into needle-like concavities. The amount of the solution 15 containing a target substance and the like per unit 13 of the microneedle array can be pre-adjusted or measured by regulating the viscosity of the target substance and the wettability with the female mold.

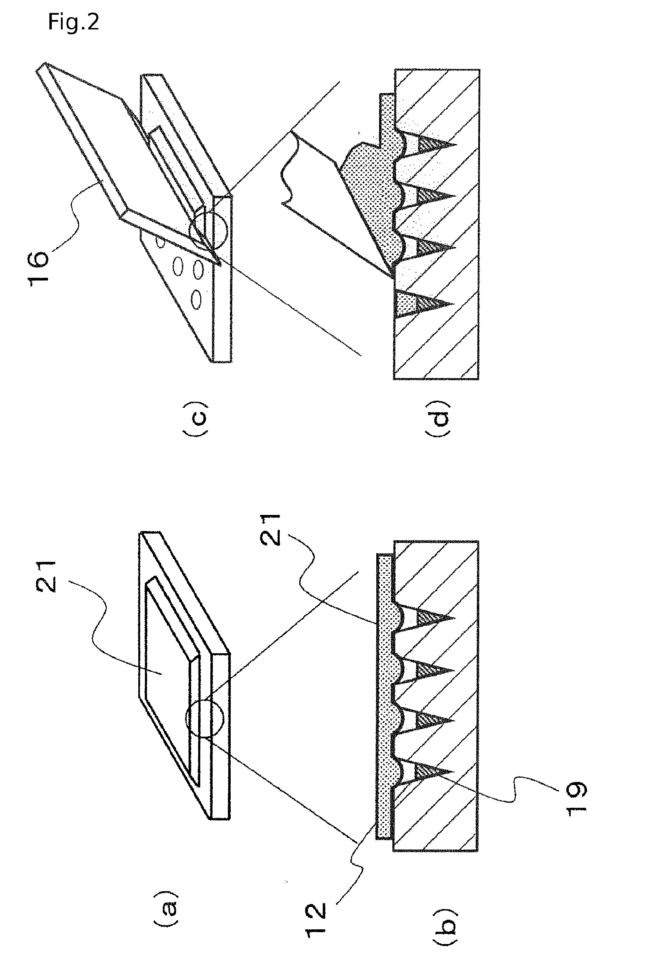

Next, the viscous solution of the thread-forming base is coated on the female mold. As illustrated in Fig. 2, since a target substance portion 19 is formed in the bottom of the concavities 12, a base 21 is injected above that.

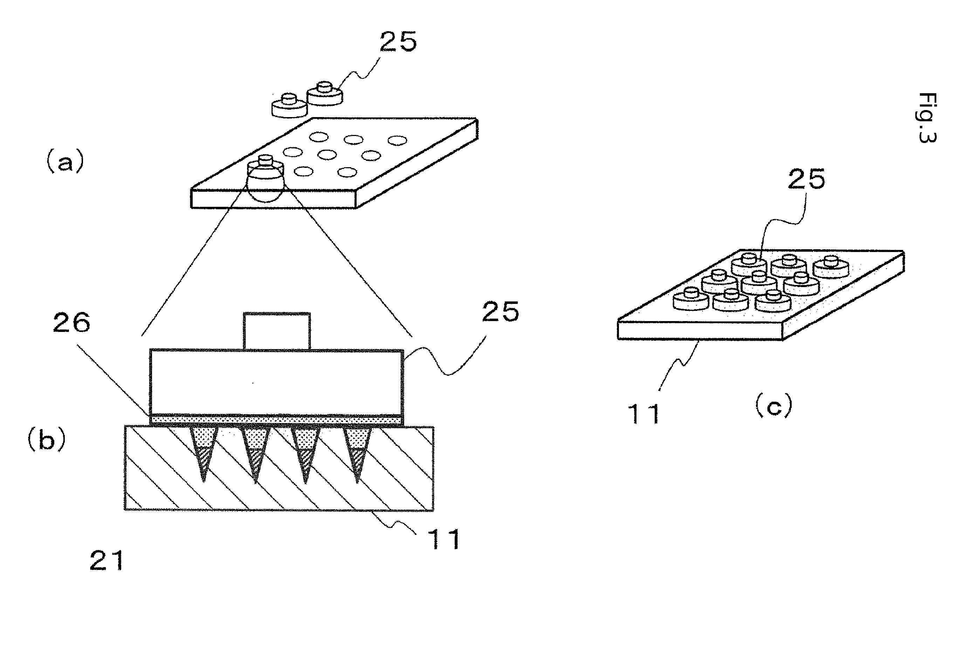

In Figs. 3 (a) and 3 (b), a substrate 25 is pressed against the female mold 11. Here, a glue 26 may be coated on the surface of the substrate 25. The glue 26 coated on the substrate 25 is a thread-forming substance similar to the base 21. It is especially preferred to use the same material as the base 21. This is to make the substrate 25 reliably adhere with the base which can be seen in the opening of the concavity hole of the female mold 11.

Next, the whole structure is dried in a state in which the substrate 25 is pressed against the female mold 11 (Fig. 3 (c)). The drying method is not specifically limited, and may be carried out by methods such as blowing air or reduced-pressure drying. However, it is preferred to not perform heating at a temperature of above room temperature, since the target substance is often a substance which is basically unstable to heat, such as a protein or a nucleic acid. This is because the target substance will suffer from denaturation or degradation. Therefore, the step of drying the whole structure may be carried out under a room temperature to low-temperature environment. Here, the term "room temperature" refers to 20°C to 30°C. Further, "low temperature" refers to a temperature of from 4°C to less than 20°C. If the target substance is stable at a given temperature, the operation efficiency increases by heating for drying.

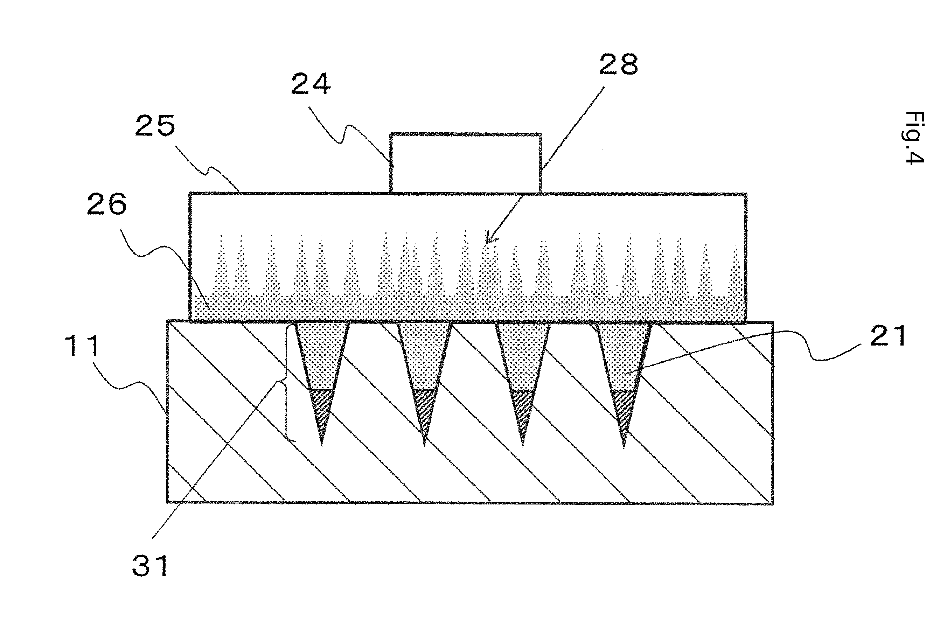

If the substrate 25 is dried while still being pressed against the female mold 11, the solvent is sucked upward from the surface of the porous substrate in contact with the female mold, and the solvent in the base and the target substance evaporates from the opposite face thereof. Specifically, the porosity of the substrate contributes to the drying and curing of the needles in the female mold. Further, as illustrated in Fig. 4, since the base 21 has a thread-forming property, the base 21 enters (28) into the pores of the substrate 25, so that the base 21 solidly fixes to the substrate 25. The reference numeral 28 in Fig. 4 represents a state in which the thread-forming base 21 has entered into the pores of the substrate 25. Specifically, along with the use of the thread-forming base 21, the porosity of the substrate 25 contributes to improving the binding force between the substrate 25 and the needles 31. By configuring in this manner, after drying and curing, when peeling the substrate 25 from the female mold 11, all of the needles 31 comprising the target substance and the base can be removed from the concavities.

Further, a protrusion 24 formed on the rear face of the substrate increases the surface area of the rear face. Specifically, the protrusion 24 provides the substrate for a microneedle array with the property of easier drying and curing.

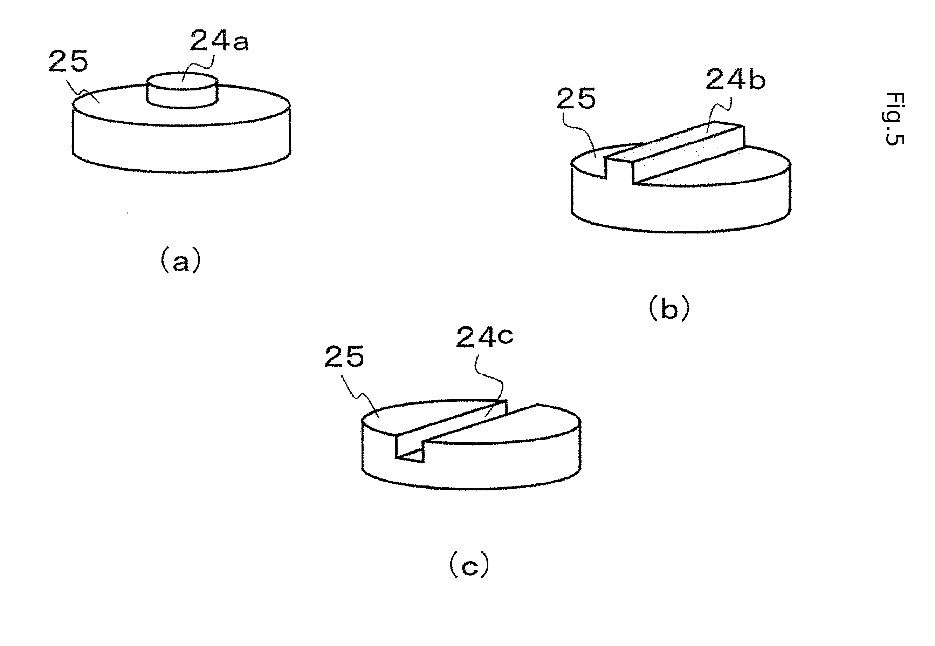

Fig. 5 illustrates examples of variations of the protrusion 24 formed on a rear face of the substrate 25. However, the present invention is not limited to these examples. Fig. 5(a) is an example in which a cylindrical protrusion 24a is formed in the center of the rear face. Fig. 5(b) is an example of a protrusion 24b with a shape in the cross-section direction. Fig. 5(c) is an example in which a concavity 24c is formed on the rear face, so that a protruding shape if formed on either side of the concavity. Thus, by forming a concavity, the protrusion 24 may also be formed as a protrusion around the periphery of a concavity.

Further, a plurality of these protrusions may be formed on the rear face of the substrate. A plurality of protrusions increases the surface area of the rear face, so that the microneedles in the female mold can be easily dried and cured.

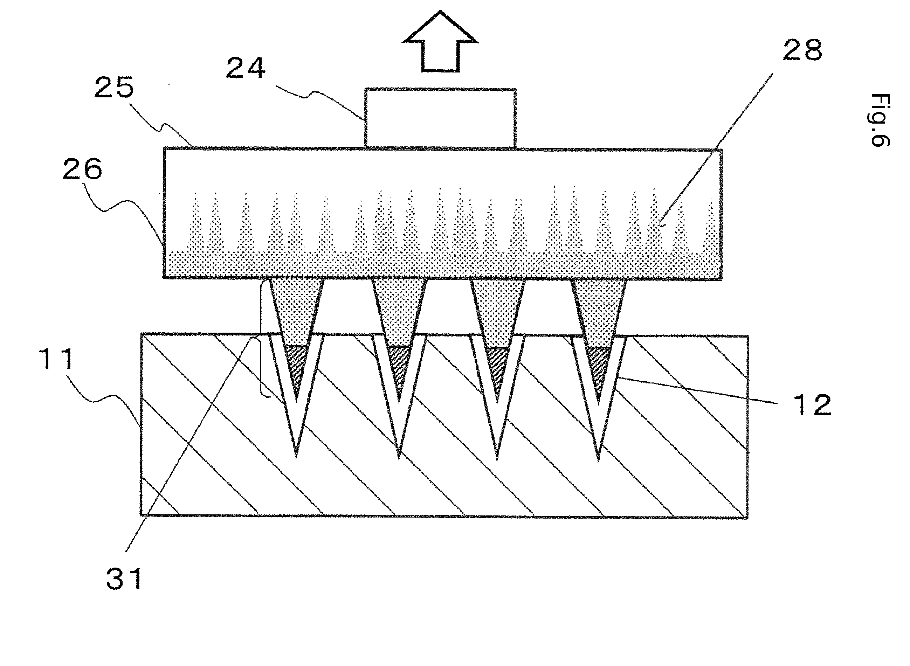

Further, the protrusion 24 on the rear face of the substrate can also be utilized as a gripping portion for lifting the substrate vertically upward when peeling the substrate from the female mold. Fig. 6 schematically illustrates a state when the microneedle array is pulled out from the female mold 11. The substrate can be lifted in a vertical direction with respect to the female mold by gripping the protrusion 24 with a manipulator and the like of the production apparatus. If the substrate is lifted at an angle to the female mold, the microneedles 31 can bump into the opening edge of concavity 12 in the female mold, and the needle portions can become damaged. By pulling out vertically, damage to the needle portions can be avoided.

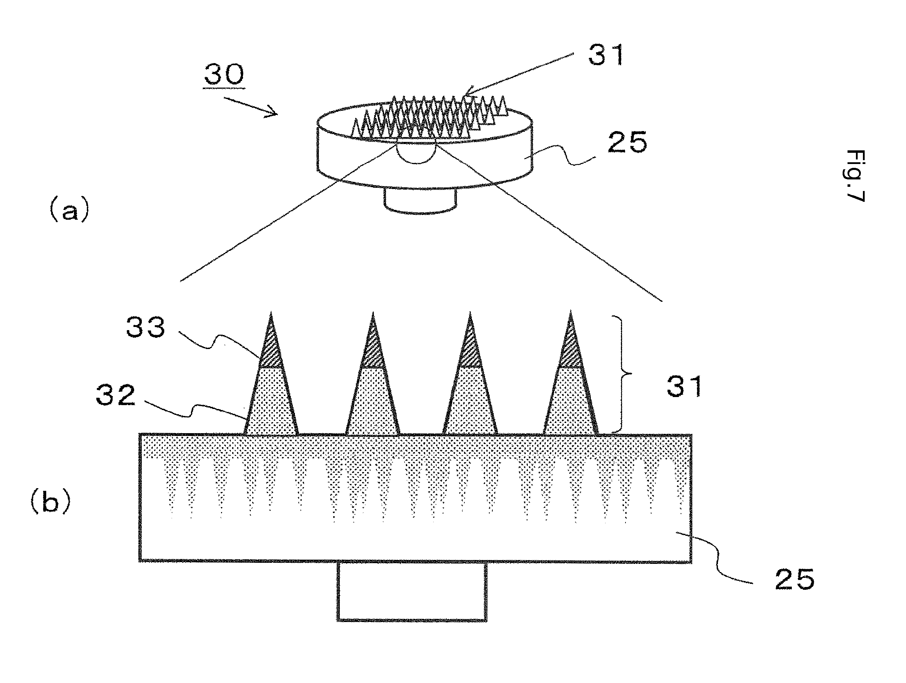

Fig. 7 illustrates an overall view (Fig. 7(a)) and a cross-sectional expanded view (Fig. 7(b)) of a microneedle array 30 which has been pulled out from the female mold. The microneedle array 30 is formed having a predetermined number of needles 21 on the substrate 25. A target substance portion 33 including a target substance is provided in the tips of the needles 31. A base portion 32 which forms the base of the needles is formed by a base made of a thread-forming polymeric substance. The target substance portion 33 is made of a target substance and the like. As already described above, examples of the target substance not only include substances having a positive effect, but also a mixture with a substance which can become a base. The base portion 32 and the substrate 25 are solidly bound as a result of an anchor effect being produced due to a part of the base diffusing into the pores of the substrate during drying and curing.

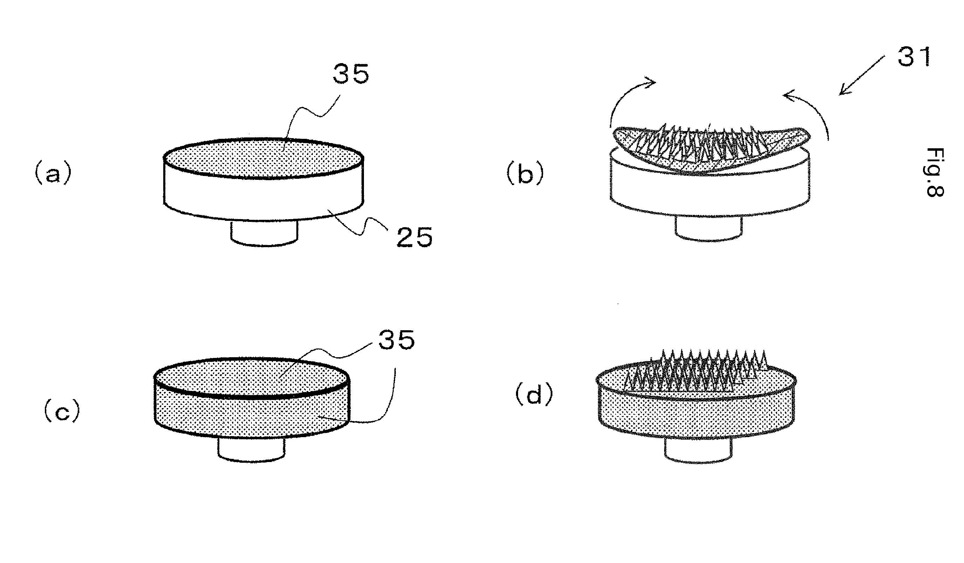

As illustrated in Figs. 8 (a) and 8(b), when a glue 35 is coated only on the surface of the substrate 25 and the substrate is then peeled from the female mold, if the drying is excessive the glue 35 contracts. This can cause a phenomenon in which a part of the substrate is warped at a peripheral portion thereof . This phenomenon especially tends to occur for a substrate produced by pressure molding of the materials. In such a case, as illustrated in Figs. 8(c) and 8(d), substrate warping can be resolved by applying the glue 35 on the side faces of the substrate as well.

The present invention will now be described using the following examples. However, these examples are in no way intended to limit the invention.

### Examples

### Example 1

Cellulose acetate for a tablet was placed in a mortar of a single punch tableting machine, and a tablet substrate having a diameter of 1.5 cm was produced at a pressing force of about 10 kN. A resin mold having, per square centimeter block, a hundred to several hundred inverted cone-shaped pores with a depth of about 500 microns and an opening diameter of about 300 microns was prepared as a female mold. Further, degassed purified water was charged into ovalbumin serving as a model antigen, hyaluronic acid, and evans blue to produce a viscous dense solution. Then, this viscous dense solution was coated onto the holes of the female mold.

Residual matter on the female mold was removed with a squeegee. Then, using a table-top centrifugal machine, the whole female mold was rotated, thereby the female mold was filled under application of a load by centrifugal force. Further, purified water was addedto sodium chondroitin sulfate to prepare a viscous dense solution. This viscous dense solution was coated on the female mold, and the cellulose acetate substrate produced by tableting machine was then covered thereon. The whole female mold was rotated using the table-top centrifugal machine to perform drying and curing. Subsequently, when the substrate was peeled from the female mold, all of the needles could be removed while stuck to the substrate, so that a microneedle array could be obtained.

### Example 2

Crystalline cellulose was placed in a single punch tableting machine, and a tablet substrate having a diameter of 1.5 cm was produced at a pressing force of about 10 kN. Then, in the same manner as in Example 1, a viscous dense solution was prepared by dissolving ovalbumin serving as a model antigen, sodium chondroitin sulfate C, and evans blue in purified water, and this viscous dense solution was coated onto the holes of the female mold. Residual matter on the female mold was removed. Then, using a table-top centrifugal machine, the whole female mold was rotated, thereby the female mold was filled under application of a load by centrifugal force.

Purified water was addedto hyaluronic acid to prepare a viscous solution. This viscous solution was coated on the female mold, and the substrate produced by tableting was covered thereon. The whole female mold was rotated while applying centrifugal force with the table-top centrifugal machine to perform drying and curing. Subsequently, when the substrate was peeled from the female mold, all of the needles could be peeled off while stuck to the substrate, so that a microneedle array was obtained.

### Example 3

Chitin was placed in a single punch tableting machine, and a tablet substrate having a diameter of 1.5 cm was produced at a pressing force of about 12 kN. Then, in the same manner as in Example 2, a viscous dense solution containing ovalbumin serving as a model antigen was coated onto the holes of the female mold. Residual matter on the female mold was removed. Then, using a table-top centrifugal machine, the whole female mold was rotated, thereby the female mold was filled under application of a load by centrifugal force.

The sodium chondroitin sulfate dense solution used in Example 1 was coated on the female mold, and the substrate was covered thereon. The whole female mold was rotated while applying centrifugal force with the table-top centrifugal machine to perform drying and curing. Subsequently, when the substrate was peeled from the female mold, all of the needles could be peeled off while stuck to the substrate, so that a microneedle array was obtained.

### Example 4

A square filter having a length of 1.5 cm and a width of 1. 5 cm was produced by cutting a porous sheet made of polyethylene. Degassed purified water was addedto insulin (produced by the inventor), evans blue, and sodium chondroitin sulfate, and the resultant solution was thoroughly dissolved and mixed to produce a viscous dense solution.

In the same manner as in Example 1, the viscous dense solution containing insulin was coated onto the holes of the female mold. Residual matter on the female mold was removed with a squeegee. Then, using a table-top centrifugal machine, the whole female mold was rotated, thereby the female mold was filled under application of a load by centrifugal force. The female mold was further centrifuged to perform drying and curing. A viscous dense solution produced by adding purified water to sodium chondroitin sulfate was coated on the female mold, and a porous substrate made of polyethylene was covered thereon. The whole female mold was rotated using the table-top centrifugal machine to perform drying and curing. Subsequently, when the substrate was peeled from the female mold, all of the needles could be pulled out while stuck to the substrate, so that a microneedle array was obtained.

### Example 5

A square filter having a length of 1.5 cm and a width of 1. 5 cm was produced by cutting a porous sheet made of poly (methyl methacrylate). Degassed purified water was addedto human growth hormone, lissamine green, and sodium chondroitin sulfate, and the resultant solution was thoroughly dissolved and mixed to produce a viscous dense solution. In the same manner as in Example 1, the viscous dense solution containing human growth hormone was coated onto the holes of the female mold. Residual matter on the female mold was removed with a squeegee. Then, using a table-top centrifugal machine, the whole female mold was rotated, thereby the female mold was filled under application of a load by centrifugal force.

The female mold was further centrifuged to perform drying and curing. A viscous dense solution produced by adding purified water into sodium chondroitin sulfate was coated on the female mold, and a porous substrate made of poly(methyl methacrylate) was covered thereon. The whole female mold was rotated while applying centrifugal force with the table-top centrifugal machine to perform drying and curing. Subsequently, when the substrate was peeled from the female mold, all of the needles could be pulled out while stuck to the substrate, so that a microneedle array was obtained.

### Example 6

A square filter having a length of 1.5 cm and a width of 1. 5 cm was produced by cutting a porous sheet made of polyvinyl chloride. Desmopressin was dissolved in a phosphate buffer having a pH of 6.5. Further, evans blue and sodium chondroitin sulfate were added and dissolved to produce a viscous dense solution. In the same manner as in Example 1, the viscous dense solution containing desmopressin was coated onto the holes of the female mold. Residual matter on the female mold was removed with a squeegee. Then, using a table-top centrifugal machine, the whole female mold was rotated, thereby the female mold was filled under application of a load by centrifugal force.

The same viscous dense solution of sodium chondroitin sulfate that was used in Example 5 was coated on the female mold, and a porous substrate made of polyvinyl chloride was covered thereon. The whole female mold was rotated while applying centrifugal force with the table-top centrifugal machine to perform drying and curing. Subsequently, when the substrate was peeled from the female mold, all of the needles could be pulled out while stuck to the substrate, so that a microneedle array could be obtained.

### Example 7

A square filter having a length of 1.5 cm and a width of 1. 5 cm was produced by cutting a porous sheet made of a chlorinated polyethylene-styrene resin. An erythropoietin injection (trade name: Espo, 24,000 IU/mL, Kyowa Hakko Kirin Co. , Ltd.) and degassed purified water were addedto evans blue and high-molecular-weight dextran, and the resultant mixture was dissolved to produce a viscous dense solution. In the same manner as in Example 1, the viscous dense solution containing erythropoietin was coated onto the holes of the female mold. Residual matter on the female mold was removed with a squeegee. Then, using a table-top centrifugal machine, the whole female mold was rotated, thereby the female mold was filled under application of a load by centrifugal force.

Degassed purified water containing polyethylene glycol 400 in a 0.1% concentration was addedto high-molecular-weight dextran to produce a viscous dense solution. This viscous dense solution was coated on the female mold, and a porous substrate made of chlorinated polyethylene-styrene resin was covered thereon. This structure was left overnight while pressing the porous substrate on the female mold to perform drying and curing. Subsequently, when the substrate was peeled from the female mold, all of the needles could be pulled out while stuck to the substrate, so that a microneedle array could be obtained.

### Comparative Example 1

In the same manner as in Example 1, a viscous dense solution produced by adding degassed purified water into ovalbumin used as a model antigen, hyaluronic acid, and evans blue was coated onto the holes of a female mold having, per square centimeter block, a hundred to several hundred inverted cone-shaped pores with a depth of about 500 microns and an opening diameter of about 300 microns. Residual matter on the female mold was removed. Then, using a table-top centrifugal machine, the whole female mold was rotated, thereby the female mold was filled under application of a load by centrifugal force.

A dense solution produced by adding purified water to sodium chondroitin sulfate was coated on the female mold, and an acrylic plate having a thickness of 3 mm and a size of 1.5 cm × 1.5 cm was covered thereon. The resultant structure was left for a whole day at room temperature which sandwiched with a clip. Alternatively, the whole female mold was rotated for 2 hours by a table-top centrifugal machine. However, in either case the drying and curing was insufficient. Although the acrylic sheet was peeled from the female mold, the microneedle array could not be released.

### Comparative Example 2

The experiment was carried out in the same manner as in Comparative Example 1, except that a sheet made of polypropylene having a thickness of 0.75 mm was used instead of the acrylic plate. However, similar to Comparative Example 1, when an attempt was made to release the microneedle array from the female mold, the microneedle array could not be released.

Comparing Examples 1 to 7 with Comparative Examples 1 and 2, when a non-porous substrate was used such as an acrylic plate or a polypropylene sheet, the needles could not be pulled out together with the substrate. This is thought to be because using a porous substrate allows the needles to be dried inside the female mold, and the thread-forming base diffuses the pores of the substrate, whereby the substrate and the needles are solidly bonded by the anchor effect.

### Example 8

Ethyl cellulose or cellulose acetate was placed in a mortar of a single punch tableting machine, and a tablet substrate having a diameter of 1.5 cm was produced at a pressing force of about 15 kN. Then, in the same manner as in Example 2, a viscous dense solution prepared by dissolving ovalbumin serving as a model antigen, sodium chondroitin sulfate, and evans blue in purified water was coated onto the holes of the female mold.

Residual matter on the female mold was removed with a squeegee. Then, using a table-top centrifugal machine, the whole female mold was rotated, thereby the female mold was filled under application of a load by centrifugal force. Purified water was addedto sodium chondroitin sulfate to prepare a viscous dense solution (glue). This viscous dense solution was coated on the female mold, and the tablet substrate produced by tableting was covered thereon. The whole female mold was dried and cured using the table-top centrifugal machine. Subsequently, when the substrate was peeled from the female mold, all of the needles could be pulled out while stuck to the substrate, so that a microneedle array could be obtained. However, as drying progressed, the peripheral portion of the tablet substrate warped.

### Example 9

A sodium chondroitin sulfate glue was coated also on the side faces of the substrate. A sample produced by coating glue on the side faces of a tablet substrate made of cellulose acetate or ethyl cellulose did not exhibit warping of a peripheral portion of the tablet substrate even when excessively dried.

Comparing Examples 8 and 9, it can be seen that by coating the glue which is to be coated on the substrate not only on the upper face but also on the side faces, the advantageous effect that warping of the finished microneedle array can be prevented is obtained.

Thus, coating a glue made from a thread-forming polymeric substance on the side faces of the substrate as well can be said to be a preferred method for avoiding substrate warping. However, if a glue made from a thread-forming polymer base is coated on the side faces of the tablet as well, this adds one step to the operation. The reason why the substrate warps and peels away from the surface is that the contraction force of the glue during drying is stronger than the binding force of the constituent materials of the substrate. Therefore, to more strongly bind the substrate constituent materials together during drying, the properties of a substrate produced by mixing a water-insoluble polymer and an adhesive polymer (also referred to as "adhesive polymeric substance") were investigated. Here, the term "adhesive polymeric substance" includes substances categorized as binders for a pharmaceutical additive.

Specifically, a carboxy vinyl polymer (trade name: Hiviswako 103 ("Hiviswako" is a registered trademark of Wako Pure Chemical Industries, Ltd.)), carboxymethyl cellulose sodium (Wako Pure Chemical Industries, Ltd.), a polyvinyl alcohol (Nacalai Tesque Inc.), and hydroxy propyl cellulose (Nippon Soda Co., Ltd.), which are adhesive polymers, were mixed into cellulose acetate and ethyl cellulose, which are water-insoluble polymers, in various blending ratios. The resultant mixtures were tableted to produce a tablet-shaped substrate.

Cellulose acetate (AC), ethyl cellulose (EC), Hiviswako 103 (HV), carboxymethyl cellulose sodium (CMC-NA), polyvinyl alcohol (PVA), and hydroxy propyl cellulose (HPC) are respectively abbreviated as AC, EC, HV, CMC-NA, PVA, and HPC.

Chondroitin sulfate (CDR: Nacalai Tesque Inc.) and dextran (DEX: Nacalai Tesque Inc., polymer) were used as the glue. The glue was coated on one face only of a tablet substrate produced by applying pressure to the above-described materials. Then, microneedle arrays were produced using a resin female mold. Each microneedle array was left for 3 weeks at room temperature under conditions of about 35% humidity, and then observed. The results are shown in Tables 1 and 2.

**[Table 1]**

| | **Glue** | 10:1 | 8,5:1.5 | 8:2 | 7,5:2.5 | 7:3 |
|---|---|---|---|---|---|---|
| AC:HV | CDR | × | ○ | ○ | ○ | ○ |
| | DEX | ○ | ○ | ○ | ○ | ○ |
| AC: CMC-NA | CDR | × | × | × | × | × |
| | DEX | ○ | ○ | ○ | ○ | ○ |
| AC:PVA | CDR | × | × | × | × | × |
| | DEX | ○ | ○ | ○ | ○ | ○ |
| AC:HPC | CDR | ○ | ○ | ○ | ○ | ○ |
| | DEX | ○ | ○ | ○ | ○ | ○ |

**[Table 2]**

| | **Glue** | 10:1 | 8.5:1.5 | 8:2 | 7.5:2.5 | 7:3 |
|---|---|---|---|---|---|---|
| EC:HV | CDR | ○ | ○ | ○ | ○ | ○ |
| | DEX | ○ | ○ | ○ | ○ | ○ |
| EC:CMC-NA | CDR | × | × | × | × | × |
| | DEX | × | × | × | × | × |
| EC:PVA | CDR | × | × | × | × | × |
| | DEX | × | ○ | ○ | ○ | ○ |
| EC:HPC | CDR | ○ | ○ | ○ | ○ | ○ |
| | DEX | ○ | ○ | ○ | ○ | ○ |

Table 1 shows the test results for substrates produced by combining cellulose acetate (AC) with Hiviswako 103 (HV), carboxymethyl cellulose sodium (CMC-NA), polyvinyl alcohol (PVA), and hydroxy propyl cellulose (HPC). For the "Glue" column, a chondroitin sulfate (CDR) column and a dextran (DEX) column are provided. The horizontal direction of the table shows the results for when the mixing ratio of cellulose acetate and the other materials was varied from 10:1 to 7:3.

In the tables, "circle" represents that a good microneedle array was obtained without any warping, and "cross" represents that warping occurred.

Table 2 shows the results for when ethyl cellulose (EC) was used instead of the cellulose acetate (AC) of Table 1.

For all of the samples, it was possible to pull out all of the needles while still stuck to the tablet substrate. Therefore, the samples in Tables 1 and 2 are all the samples according to the present invention. However, for some compositions the periphery warped even when a water-insoluble polymer and an adhesive polymer were mixed to improve warping of the substrate periphery. Specifically, carboxymethyl cellulose sodium (CMC-NA) and polyvinyl alcohol (PVA) could not overcome the drying contraction force of chondroitin sulfate (CDR) even if they were mixed with cellulose acetate (AC) or mixed with ethyl cellulose (EC).

Especially, the combination of ethyl cellulose (EC) and carboxymethyl cellulose sodium (CMC-NA) could not overcome the drying contraction force of dextran (DEX). Specifically, when a substrate is produced using these materials, it is necessary to select a glue having less contraction, or employ a method in which the tablet substrate is more strongly solidified. When a filter made of a porous polymer resin or a porous metal filter is used as the substrate, there is no warping of the substrate periphery.

However, for other combinations, generally, an effect from adding the adhesive polymer was seen. Specifically, a good microneedle array free from periphery warping could be obtained even when the glue was coated on only one face of the substrate.

### Example 10

A viscous dense solution produced by adding degassed purified water to ovalbumin used as a model antigen, sodium chondroitin sulfate, and evans blue was coated onto the holes of a resin substrate having, per square centimeter block, a hundred to several hundred inverted cone-shaped pores with a depth of about 500 microns and an opening diameter of about 300 microns.

Residual matter on the female mold was removed with a squeegee. Then, using a table-top centrifugal machine, the whole female mold was rotated, thereby the female mold was filled under application of a load by centrifugal force. A viscous dense solution produced by adding purified water to sodium chondroitin sulfate was coated on the female mold, and a porous metal filter (substrate) was covered thereon. The whole female mold was rotated by a table-top centrifugal machine to perform drying and curing. Subsequently, when the substrate was peeled from the female mold, all of the needles could be pulled out while stuck to the substrate, so that a microneedle array could be obtained.

### INDUSTRIAL APPLICABILITY

If a water-soluble thread-forming polymer is used as a base, unless a base dense viscous solution containing a macromolecular drug such as a vaccine antigen and insulin in a dissolved or suspended state followed by filling a base dense viscous solution (glue) is dried and cured while still inserted in the female mold of a microneedle array, a microneedle array for a pharmaceutical preparation cannot be produced. In order to dry and cure a base including a target substance in a female mold for a microneedle array and stronglybind to the microneedle array to a substrate, it is important that the substrate promotes adsorption and evaporation of the solvent. To achieve this, there is a need for a substrate which has excellent solvent adsorption and permeability, and which meets the various grades as a pharmaceutical preparation, such as sterility.

According to the present invention, a viscous dense solution of a base containing a target substance can be dried and cured in the female mold of a microneedle array, thereby enabling a microneedle array suited for applications such as a pharmaceutical and a cosmetic to be efficiently produced.

### DESCRIPTION OF THE REFERENCE NUMERALS

- 11: female mold
- 12: concavities formed in female mold
- 13: one unit of microneedle array on female mold
- 15: solution containing target substance and the like
- 16: squeegee
- 19: target substance injected into the bottom of concavity
- 21: base
- 24: protrusion
- 25: substrate
- 26: glue
- 30: microneedle array
- 31: needle
- 32: base portion
- 33: target substance portion

## Claims

1. A substrate for a microneedle array having at least one flat face, comprising
a protrusion on an opposite face of the flat face, and wherein
the substrate is formed from a porous material which has a porosity of which direction is directed from the flat face to the opposite face of the flat face.

2. The substrate for a microneedle array according to claim 1, comprising a water-insoluble fibrous polymer and an adhesive polymer.

3. The substrate for a microneedle array according to claim 2, wherein the fibrous polymer includes at least one of crystalline cellulose, ethyl cellulose, cellulose acetate and derivatives thereof, and chitin and derivatives thereof.

4. The substrate for a microneedle array according to any one of claims 2 and 3, wherein the adhesive polymer includes at least one of a carboxy vinyl polymer, a carboxymethyl cellulose sodium, a polyvinyl alcohol, and a hydroxy propyl cellulose.

5. The substrate for a microneedle array according to claim 1, wherein the porous material includes a porous polymer resin or a porous metal.

6. The substrate for a microneedle array according to claim 5, wherein the porous polymer resin includes at least one of polyethylene, poly(methyl methacrylate), polyvinyl chloride, acrylonitrile, chlorinated polyethylene-styrene, polyoxyethylene, and polypropylene.

7. A microneedle array, comprising:
a porous substrate having a flat face and a protrusion on an opposite face of the flat face;
a protruding base portion made of a thread-forming polymer material which is fixed to the substrate; and
a target substance portion provided on a acral portion of the protruding base.

8. The microneedle array according to claim 7, wherein the substrate includes a water-insoluble fibrous polymer and an adhesive polymer.

9. The microneedle array according to claim 8, wherein the fibrous polymer includes at least one of crystalline cellulose, ethyl cellulose, cellulose acetate and derivatives thereof, and chitin and derivatives thereof.

10. The microneedle array according to any one of claims 8 and 9, wherein the adhesive polymer includes at least one of a carboxy vinyl polymer, a carboxymethyl cellulose sodium, a polyvinyl alcohol, and a hydroxy propyl cellulose.

11. The microneedle array according to claim 8, wherein the porous material includes a porous polymer resin or a porous metal.

12. The substrate for a microneedle array according to claim 11, wherein the porous polymer resin includes at least one of polyethylene, poly(methyl methacrylate), polyvinyl chloride, acrylonitrile, chlorinated polyethylene-styrene, polyoxyethylene, and polypropylene.

13. A method for producing a microneedle array, comprising the steps of:
filling a target substance into a female mold;
further filling a base into the female mold;
pressing a porous substrate against the female mold to dry and cure; and
peeling the substrate from the female mold.

14. The method for producing a microneedle array according to claim 13, wherein the step of peeling the substrate is a step of vertically pulling the substrate out from the female mold.

15. The method for producing a microneedle array according to any one of claims 13 and 14, further comprising a step of coating a glue on the substrate before the step of pressing the substrate against the female mold to dry and cure.

16. The method for producing a microneedle array according to claim 15, wherein the step of coating a glue on the substrate include a step of coating a glue on side faces of the substrate.

17. The method for producing a microneedle array according to any one of claims 13 to 16, wherein the step of pressing the substrate against the female mold to dry and cure is carried out under a reduced-pressure environment.

18. The method for producing a microneedle array according to any one of claims 13 to 16, wherein the step of pressing the substrate against the female mold to dry and cure is carried out under a room temperature to low-temperature environment.
